# FASCICULE DE BREVET EUROPEEN

(11) **EP 1 958 605 B1**
(45) Date de publication et mention de la délivrance du brevet: **21.11.2018**
(21) Numéro de dépôt: 08290047.3
(22) Date de dépôt: 21.01.2008
(51) Int. Cl.: A61F 13/53, A61F 13/472, A61F 13/49

(54) **Procédé et dispositif d'imprégnation de produit réalisé par découpe d'une nappe continue**
Verfahren und Vorrichtung zur Imprägnierung eines Produkts, das durch Zuschnitt eines Endlosvlieses entsteht
Method and device for impregnating a product made by cutting a continuous sheet

(30) Priorité: 13.02.2007 FR 0701035
(43) Date de publication de la demande: 20.08.2008
(73) Titulaire: Essity Operations France, 93400 Saint-Ouen (FR)
(72) Inventeur: Ruppel, Rémy, 68320 Durrenentzen (FR)
(74) Mandataire: Essity Hygiene and Health AB

(56) Documents cités:
- WO-A-97/27032
- US-A- 1 555 238
- US-A- 3 183 910

## Description

L'invention concerne un nouveau procédé et un nouveau dispositif d'imprégnation de produit réalisé par découpe d'une nappe continue de matériau fibreux.

Dans le domaine des produits en matériau fibreux, il est souvent utile d'effectuer une découpe d'une série de formats individuels à l'intérieur d'une nappe continue.

En effet, le fait de découper une nappe en continu permet d'accroître les rendements de production, la vitesse d'exécution étant grandement supérieure à celle d'une découpe en feuille à feuille.

De plus, cela facilite également le processus de traitement en aval des produits découpés, en particulier leur emballage ou leur imprégnation éventuelle.

Ce procédé nécessite généralement de mettre en oeuvre un appareil spécifique, adapté aux dimensions et aux caractéristiques de la nappe à découper, ainsi qu'au format des produits découpés à obtenir.

On utilise en outre des appareils additionnels pour effectuer les autres étapes de traitement, chacun desdits appareils opérant selon le cas soit sur la nappe continue, soit sur chaque produit individuel découpé.

Or, dans le cas de produits découpés et imprégnés, on constate que les procédés et les dispositifs actuellement disponibles à ce niveau présentent un certain nombre d'inconvénients lesquels s'avèrent incompatibles avec une rentabilité accrue et une bonne qualité de produits.

Un art antérieur consiste notamment à imprégner la nappe continue avant l'étape de découpe.

L'inconvénient de ce procédé est que l'imprégnation se fait de façon homogène sur toute la surface de la nappe y compris dans les zones retirées de la nappe une fois l'étape de découpe effectuée.

Il en résulte une consommation inutile de produit d'imprégnation.

Un autre art antérieur consiste à imprégner individuellement chaque zone découpée de la nappe en aval de l'étape de découpe.

L'inconvénient de cette solution est la difficulté à appliquer le produit d'imprégnation exactement à l'emplacement de la zone découpée.

Il en résulte souvent une imprégnation incomplète et aléatoire des produits au final.

Un autre inconvénient de ces solutions est la nécessité de prévoir deux appareils distincts pour effectuer la découpe et l'imprégnation.

L'invention vise donc à proposer une solution au problème de l'imprégnation des produits réalisés par découpe d'une nappe continue, laquelle ne présente pas les inconvénients de l'art antérieur susmentionné.

A cet effet, et conformément à l'invention, il est proposé un procédé d'imprégnation de produit en matériau fibreux formé à partir d'une nappe continue, le procédé comprenant au moins une première étape de découpe d'une zone spécifique de ladite bande continue et au moins une seconde étape d'application d'un produit d'imprégnation sur ladite zone spécifique, le procédé étant remarquable en ce que les première et seconde étapes sont effectuées de manière simultanée ou quasi simultanée.

Dans une variante avantageuse du procédé de l'invention, les première et seconde étapes sont effectuées au moins partiellement au moyen d'un même appareil.

Dans une autre variante du procédé de l'invention, la nappe continue est formée d'un matériau non tissé, en particulier une nappe de coton.

Dans une autre variante du procédé de l'invention, la nappe continue est formée d'un matériau cellulosique, en particulier une feuille de papier.

Dans une autre variante du procédé de l'invention, le produit d'imprégnation est un liquide.

Dans une autre variante du procédé de l'invention, le produit d'imprégnation est une pâte.

Dans une autre variante du procédé de l'invention, le produit obtenu est un tampon de coton à démaquiller ou une lingette humide.

Dans une autre variante du procédé de l'invention, le produit obtenu est une serviette périodique ou une couche bébé ou une couche adulte

L'invention porte également sur un dispositif destiné à la mise en oeuvre du procédé susmentionné, le dispositif étant remarquable en ce qu'il comprend au moins un premier moyen de découpe d'une zone spécifique d'une nappe continue formée en un matériau fibreux et au moins un second moyen pour appliquer un produit d'imprégnation sur ladite zone spécifique, les premier et second moyens étant monobloc.

Dans une variante avantageuse du dispositif de l'invention, le second moyen est disposé relativement au premier moyen de manière à se trouver en contact ou en face de la zone spécifique au moment de la découpe.

Dans une autre variante du dispositif de l'invention, le premier moyen est constitué d'un rouleau muni d'un motif en creux, ou en relief, dont les bords sont coupants et délimitent globalement la zone spécifique à découper, la nappe continue étant pressée entre ce rouleau et un rouleau lisse, dit rouleau enclume, de manière à opérer la découpe.

Dans une autre variante du dispositif de l'invention, le premier moyen est constitué d'une lame montée sur un support.

Dans une autre variante du dispositif de l'invention, la lame est mobile dans le support.

Dans une autre variante du dispositif de l'invention, le support est courbe, et, de préférence, cylindrique.

Dans une autre variante du dispositif de l'invention, le support est plan.

Dans une autre variante du dispositif de l'invention, le premier moyen est un poinçon, la nappe continue étant pressée entre ce poinçon et une matrice percée d'au moins un trou dont les dimensions correspondent à celles de la zone spécifique à découper de manière à opérer la découpe.

Dans une autre variante du dispositif de l'invention, le second moyen comprend une buse apte à projeter le produit d'imprégnation en direction de la nappe continue.

Dans une autre variante du dispositif de l'invention, le second moyen comprend un corps absorbant apte à contenir une certaine quantité de produit d'imprégnation et à transférer au moins une partie dudit produit sur la nappe lorsqu'il est pressé contre celle-ci.

Dans une autre variante du dispositif de l'invention, le second moyen comprend un corps non absorbant apte à retenir une certaine quantité de produit d'imprégnation à sa surface et à transférer au moins une partie dudit produit sur la nappe lorsqu'il est mis en contact avec celle-ci.

Telle que configurée, l'invention permettra donc de déposer le produit d'imprégnation uniquement sur les zones découpées de la nappe continue, évitant ainsi une surconsommation inutile de produit.

En outre, la découpe et l'imprégnation se faisant au cours d'une étape commune de traitement et/ou en utilisant un appareil unique, il en résultera un gain de temps et de place avantageux pour la productivité et l'efficacité globale du procédé de fabrication des produits finaux.

D'autres avantages et caractéristiques ressortiront mieux de la description qui va suivre d'un procédé et d'un dispositif selon l'invention, en référence aux dessins sur lesquels :
- la figure 1 représente une vue schématique d'un dispositif d'imprégnation conforme à l'invention,
- les figures 2A, 2B et 2C représentent des vues en perspective d'inserts de découpe pouvant être utilisés dans le dispositif représenté à la figure 1,
- la figure 3 représente de façon schématique une variante de réalisation d'un dispositif d'imprégnation conforme à l'invention.

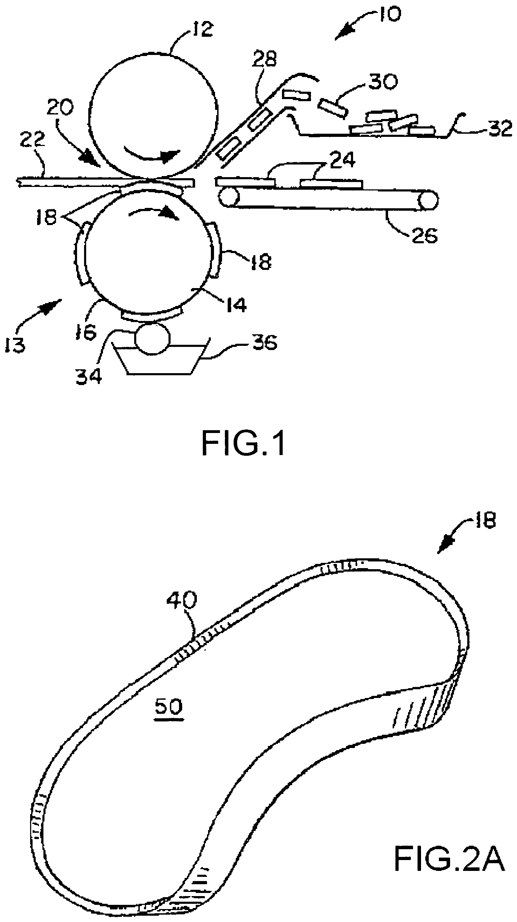

En référence à la figure 1, il est représenté un appareil de découpe 10 rotatif comprenant un rouleau enclume 12 coopérant avec un rouleau de découpe 13. Le rouleau enclume 12 peut être formé en un métal ferrique ou non ferrique et posséder une surface lisse. Le rouleau enclume 12 peut également être formé d'un matériau compressible, tel que du néoprène.

Même si sur la figure représentée, le rouleau enclume 12 est disposé au-dessus du rouleau de découpe 13, il sera toutefois préférable de disposer le rouleau de découpe 13 au-dessus du rouleau enclume 12, de manière à favoriser le transfert du produit d'imprégnation par gravité.

Le rouleau de découpe 13 comprend un support cylindrique 14 muni d'une circonférence externe 16 et au moins un insert de découpe 18 monté sur le support cylindrique 14.

L'insert 18 pourra être fixé sur le support 14 au moyen d'un boulon de manière à permettre son remplacement rapide durant les phases de nettoyage ou de modification du format de découpe.

Le rouleau de découpe 13 sera avantageusement formé en un métal rigide, par exemple en acier, de manière à éviter les déformations de sa surface externe au cours de son utilisation.

Le rouleau de découpe 13 pourra avantageusement posséder plusieurs inserts de découpe 18 répartis de manière régulière à la circonférence 16 du support 14, les inserts 18 décrivant de préférence un cercle autour de l'axe du support 14 et étant espacés d'un angle constant.

Dans l'exemple représenté à la figure 1, le rouleau de découpe 13 comporte quatre inserts 18 espacés de manière égale sur la circonférence externe 16 du support 14, les inserts 18 étant disposés en cercle autour de l'axe du support 14.

Le rouleau de découpe 13 et le rouleau enclume 12 effectuent une rotation en sens inverse et coopèrent pour former une zone de pincement 20 à travers laquelle passe une bande 22 de matériau fibreux, du type non tissé, comme une nappe de coton par exemple, ou du type cellulosique, comme une feuille de papier par exemple.

Au moment où elle passe à travers la zone de pincement 20, la bande 22 est pressée entre un des inserts 18 du rouleau de découpe 13 et la surface lisse du rouleau enclume 12 ce qui provoque la découpe de la bande 22 en articles individuels 24. La découpe se faisant sensiblement le long des bords externes des inserts 18, chacun des articles 24 obtenus a donc la forme de l'insert 18 utilisé.

Lors de cette opération de découpe, il se produit également un dépôt de produit d'imprégnation sur la face de l'article 24 qui était en contact avec l'insert 18.

Cette imprégnation se produit quasi simultanément, et, de préférence, de façon simultanée, avec la découpe de l'article 24.

Elle pourra notamment résulter de l'action d'un moyen d'application de produit d'imprégnation intégré au rouleau de découpe 13, ledit moyen d'application étant avantageusement disposé dans une zone couverte par le ou les inserts 18.

Ainsi, lorsque la bande 22 passe dans la zone de pincement 20, c'est-à-dire en face et/ou au contact de la zone du rouleau de découpe 13 couverte par l'insert 18, elle se trouve normalement dans la zone d'action dudit moyen d'application et est donc susceptible de recevoir une quantité de produit d'imprégnation de la part de celui-ci.

Une fois découpés et imprégnés, les articles 24 sont transportés par des moyens conventionnels, tels qu'un tapis transporteur 26, vers un emplacement où ils peuvent être empilés, emballés et par la suite expédiés en palettes par exemple.

Les déchets de bande 30 restant après l'opération de découpe peuvent, quant à eux, être dirigés hors de la zone de pincement 20 vers une conduite 28 en utilisant un moyen d'aspiration, de soufflage, la gravité ou d'autres moyens mécaniques conventionnels. Ces déchets 30 sont alors collectés dans un bac de décharge 32 avant d'être recyclés ou détruits.

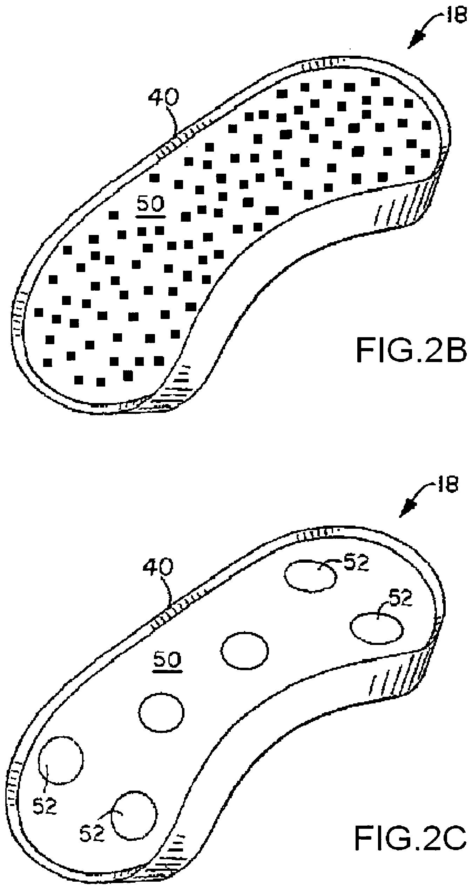

En référence aux figures 2A, 2B et 2C, il est représenté des exemples d'inserts utilisables dans le cadre du dispositif représenté à la figure 1.

Ces inserts 18 sont adaptés, de part leur forme, à effectuer la découpe de serviettes hygiéniques. Il est évident que la forme desdits inserts 18 pourra être adaptée en fonction des articles à former. En particulier, il sera envisageable d'utiliser des inserts 18 de forme ronde, ovale, carrée, rectangulaire pour former des tampons de coton à démaquiller.

Ces inserts 18 possèdent également une courbure de manière à s'adapter à la courbure du support cylindrique 14 sur lesquels ils doivent être fixés.

Chacun d'eux possède un bord coupant 40 sur son pourtour de manière à effectuer la découpe de la bande continue 22 lorsqu'il presse celle-ci contre le rouleau enclume 12 dans la zone de pincement 20 mentionnée précédemment. Ce bord coupant 40 pourra être continu, comme dans les exemples représentés, ou discontinu afin de former des prédécoupes. Il pourra également comporter une série de dents sur la totalité ou une partie de sa longueur.

Le bord coupant 40 délimite une paroi interne 50 de l'insert.

Cette paroi interne 50 possédera de préférence une hauteur inférieure à celle du bord coupant 40 de manière à être disposée en retrait par rapport à celui-ci.

Dans les modes de réalisation représentés sur les figures 2A, 2B et 2C, cette paroi interne 50 constitue en outre un moyen d'application d'un produit, liquide ou pâte, sur la bande continue.

Dans l'exemple de la figure 2A, cette paroi interne 50 est sensiblement lisse et possède des propriétés de surface la rendant apte à retenir un liquide ou une pâte sous forme d'une couche plus ou moins fine à sa surface et à déposer au moins une partie dudit liquide ou de ladite pâte sur la bande continue 22 lorsqu'elles sont mises en contact, notamment dans la zone de pincement 20. Le liquide ou la pâte aura été déposé préalablement sur la paroi interne 50, notamment avant l'étape de découpe, en utilisant tout moyen conventionnel à ce niveau. On pourra par exemple disposer dans le dispositif représenté sur la figure 1, en amont de la zone de pincement 20, un rouleau d'enduction 34 additionnel, lequel sera configuré pour déposer une couche de liquide ou de pâte sur la paroi interne 50 de l'insert 18. Ce rouleau d'enduction 34 sera avantageusement constitué d'une matière compressible de façon à être facilement pressé contre l'insert. Il sera également constitué d'une matière absorbante de manière à contenir une certaine quantité de liquide ou de pâte en son sein et à transférer par la suite plus ou moins de ce liquide ou de cette pâte sur l'insert 18 en fonction de la pression qu'il exerce sur l'insert 18. Le liquide ou la pâte pourra notamment provenir d'un récipient 36 dans lequel le rouleau d'enduction 34 sera partiellement immergé.

Il est également envisageable de remplacer ce rouleau d'enduction 34 par une bande de feutre partiellement immergée dans un liquide ou imprégnée d'un liquide ou d'une pâte et en contact ponctuel avec le rouleau de découpe 13 de manière à enduire les inserts 18, au moins en partie, de liquide ou de pâte.

Dans l'exemple de la figure 2B, la paroi interne 50 est formée d'un matériau compressible et absorbant, lequel pourra posséder des caractéristiques sensiblement similaires à celles du matériau utilisé pour former le rouleau d'enduction 34 mentionné précédemment.

Ladite paroi 50 contiendra en son sein le liquide ou la pâte destiné à imprégner la bande continue 22, ledit liquide ou ladite pâte ayant été préalablement appliqué sur ladite paroi 50 par tout moyen conventionnel. On pourra notamment envisager de faire passer les inserts 18 du rouleau de découpe 13 de la figure 1 à travers un bac rempli dudit liquide ou de ladite pâte, en amont de la zone de pincement 20, de manière à en imprégner les parois internes 50 desdits inserts 18. On pourra également prévoir d'installer un réservoir de liquide ou de pâte à l'intérieur du support cylindrique 14 du rouleau de découpe 13, ledit réservoir étant en communication directe ou indirecte avec ladite paroi interne 50.

Dans l'exemple de la figure 2C, la paroi interne 50 est percée d'une série de trous 52, chacun des trous 52 étant disposé en face et dans le prolongement d'une buse d'éjection de liquide ou de pâte, non représentée, fixée à l'intérieur de ladite paroi 50 ou intégrée dans le support cylindrique 14 du rouleau de découpe 13.

En synchronisant l'étape d'éjection du liquide ou de la pâte avec l'étape de découpe, notamment en actionnant les buses au moment où les trous 52 font face à la zone 24 de la bande à découper, c'est-à-dire lorsque l'insert 18 se trouve dans la zone de pincement 20, il est possible d'appliquer le liquide ou la pâte uniquement dans la zone à découper 24 et de manière simultanée ou quasi simultanée avec la découpe.

En outre, on pourra prévoir d'alimenter les buses au moyen de conduits d'alimentation reliés à un ou plusieurs réservoirs internes ou externes au rouleau de découpe, chacun des réservoirs pouvant contenir un liquide ou une pâte particulière.

Dans une variante de réalisation non représentée, il est envisageable de remplacer les buses d'éjection par tout système de projection de liquide. En particulier, on pourra prévoir un espace interne à l'intérieur du support cylindrique 14, dans lequel une pression interne sera créée de manière à extruder le produit d'imprégnation à travers les trous 52 de la paroi interne 50. Par ailleurs, ladite zone interne 50 sera avantageusement formée dans un matériau compressible et absorbant de manière à restituer du produit même sur les parties de la bande continue 22 qui ne sont pas disposées en face ou à proximité des trous 52, la quantité de produit appliquée sur ces parties étant toutefois plus faible que celle fournie directement à travers les trous 52.

Les trous 52 pourront être répartis de manière uniforme sur toute la surface de la paroi interne 50. Toutefois, il sera avantageux de les disposer sous forme de cercles concentriques, les trous 52 des cercles les plus proches du centre possédant un diamètre plus gros que les trous 52 des cercles les plus proches de la périphérie, de manière à appliquer plus de produit dans la partie centrale de la zone découpée 24 que sur les bords.

Dans une autre variante de réalisation non représentée, il sera également envisageable de rendre le bord coupant mobile par rapport au support cylindrique.

Il sera ainsi possible de déplacer ce bord coupant d'une position de repos, où il sera positionné sous la surface extérieure de la paroi interne susmentionnée, à une position active de découpe, où il sera en saillie par rapport à cette même surface.

Ce déplacement pourra se faire au moment où la paroi interne fera face à la bande à découper dans la zone de pincement. Il pourra notamment être provoqué par l'action d'une came disposée à l'intérieur du rouleau de découpe et excentrée par rapport à l'axe du rouleau, laquelle agira sur un autre des bords du couteau ou de la lame portant le bord coupant.

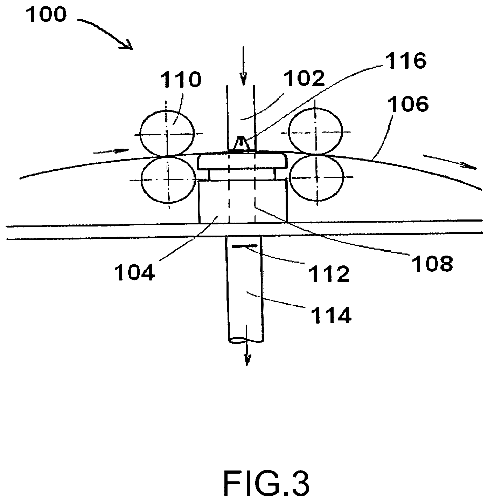

En référence à la figure 3, il est représenté une variante de réalisation d'un dispositif de découpe conforme à l'invention.

Le dispositif de découpe 100 se compose d'un poinçon 102 coopérant avec une matrice 104 entre lesquels circule une nappe 106 de matériau fibreux entraînée selon un mouvement pas-à-pas à une certaine fréquence.

La matrice 104 comporte une ouverture centrale 108 apte à recevoir le poinçon 102, la forme de l'ouverture 108 définissant le motif de découpe.

Pour régler la position de la nappe 106 par rapport à la matrice 104 et au poinçon 102, on la fait passer entre des rouleaux d'entraînement 110, préalablement et postérieurement à la découpe.

Selon une fréquence donnée, correspondant sensiblement à celle du mouvement de la nappe 106, on déplace brutalement le poinçon 102 à travers l'ouverture 108 de la matrice 104 de manière à provoquer la découpe de la nappe 106.

Une rondelle 112 de matériau possédant la forme de l'ouverture 108 tombe alors, sous l'effet de son poids, dans un organe tubulaire 114 disposé sous la matrice 104 et se trouve ensuite recueillie dans un sachet non représenté placé sous l'organe tubulaire 114.

Pour permettre l'imprégnation simultanée des rondelles 112 découpées, on équipe le poinçon 102 d'une ou plusieurs buses d'éjection 116 disposées à l'intérieur du poinçon 102 et communiquant avec la face du poinçon 102 destinée à percuter la nappe 106, la ou les buses 116 étant alimentées en produit d'imprégnation à partir d'un réservoir interne ou externe et en utilisant des moyens conventionnels.

Dans une autre variante de l'invention, il est également envisageable d'utiliser, comme moyen d'application du produit à imprégner, la surface du poinçon 102 destinée à percuter la nappe 106. Celle-ci possédera donc des propriétés de surface la rendant apte à retenir ledit produit jusqu'à son transfert par contact sur la nappe 106. Tout moyen conventionnel pourra par ailleurs être utilisé pour déposer ledit produit sur ladite surface préalablement à l'étape de découpe.

Il est bien entendu que les exemples de réalisation donnés ci-dessous ne sont nullement limitatifs pour la présente invention, d'autres modifications ou ajouts dans les étapes du procédé ou dans les moyens mis en oeuvre au niveau du dispositif pouvant être envisagés sans sortir du cadre de l'invention.

Par ailleurs, les articles obtenus par le procédé d'imprégnation de la présente invention pourront, selon le cas, être soit secs, soit humides en fonction de la quantité de produit appliqué.

## Revendications

1. Procédé d'imprégnation de produit en matériau fibreux formé à partir d'une nappe continue (22,106), le procédé comprenant au moins une première étape de découpe d'une zone spécifique (24,112) de ladite bande continue (22,106) et au moins une seconde étape d'application d'un produit d'imprégnation sur ladite zone spécifique (24,112), **caractérisé en ce que** les première et seconde étapes sont effectuées de manière simultanée.

2. Procédé selon la revendication précédente, **caractérisé en ce que** les première et seconde étapes sont effectuées au moins partiellement au moyen d'un même appareil (13,102).

3. Procédé selon la revendication 1 ou 2, **caractérisé en ce que** la nappe continue (22,106) est formée d'un matériau non tissé, en particulier une nappe de coton.

4. Procédé selon la revendication 1 ou 2, **caractérisé en ce que** la nappe continue (22,106) est formée d'un matériau cellulosique, en particulier une feuille de papier.

5. Procédé selon l'une quelconque des revendications précédentes, **caractérisé en ce que** le produit d'imprégnation est un liquide.

6. Procédé selon l'une quelconque des revendications 1 à 4, **caractérisé en ce que** le produit d'imprégnation est une pâte.

7. Dispositif (10,100) destiné à la mise en oeuvre du procédé selon l'une quelconque des revendications 1 à 6, **caractérisé en ce qu'**il comprend au moins un premier moyen (13,102) de découpe d'une zone spécifique (24,112) d'une nappe continue (22,106) formée en un matériau fibreux, au moins un second moyen (50,116) pour appliquer un produit d'imprégnation sur ladite zone spécifique (24,112), les premier (13,102) et second moyens (50,116) étant monobloc.

8. Dispositif (10,100) selon la revendication précédente, **caractérisé en ce que** le second moyen (50,116) est disposé relativement au premier moyen (13,102) de manière à se trouver en contact ou en face de la zone spécifique (24,112) au moment de la découpe.

9. Dispositif (10) selon l'une quelconque des revendications 7 et 8, **caractérisé en ce que** le premier moyen (13) est constitué d'un rouleau muni d'un motif (18) en creux, ou en relief, dont les bords (40) sont coupants et délimitent globalement la zone spécifique (24) à découper, la nappe continue (22) étant pressée entre ce rouleau (13) et un rouleau lisse (12), dit rouleau enclume, de manière à opérer la découpe.

10. Dispositif (10) selon l'une quelconque des revendications 7 et 8, **caractérisé en ce que** le premier moyen est constitué d'une lame montée sur un support (14).

11. Dispositif (10) selon la revendication précédente, **caractérisé en ce que** la lame est mobile dans le support (14).

12. Dispositif (10) selon l'une quelconque des revendications 10 et 11, **caractérisé en ce que** le support (14) est courbe, et, de préférence, cylindrique.

13. Dispositif selon l'une quelconque des revendications 10 et 11, **caractérisé en ce que** le support est plan.

14. Dispositif (100) selon l'une quelconque des revendications 7 et 8, **caractérisé en ce que** le premier moyen (102) est un poinçon, la nappe continue (106) étant pressée entre ce poinçon (102) et une matrice (104) percée d'au moins un trou (108) dont les dimensions correspondent à celles de la zone spécifique (112) à découper de manière à opérer la découpe.

15. Dispositif (10,100) selon l'une quelconque des revendications 7 à 14, **caractérisé en ce que** le second moyen (50,116) comprend une buse apte à projeter le produit d'imprégnation en direction de la nappe continue (22,106).

16. Dispositif (10) selon l'une quelconque des revendications 7 à 14, **caractérisé en ce que** le second moyen (50) comprend un corps absorbant apte à contenir une certaine quantité de produit d'imprégnation et à transférer au moins une partie dudit produit sur la nappe (22) lorsqu'il est pressé contre celle-ci.

17. Dispositif (10,100) selon l'une quelconque des revendications 7 à 14, **caractérisé en ce que** le second moyen (50,102) comprend un corps non absorbant apte à retenir une certaine quantité de produit d'imprégnation à sa surface et à transférer au moins une partie dudit produit sur la nappe (22,106) lorsqu'il est mis en contact avec celle-ci.

## Patentansprüche

1. Verfahren zum Imprägnieren eines aus einer Endlosbahn (22, 106) gebildeten Fasermaterialprodukts, wobei das Verfahren wenigstens einen ersten Schritt zum Zuschneiden eines spezifischen Bereichs (24, 112) des Endlosbands (22, 106) und wenigstens einen zweiten Schritt zum Aufbringen eines Imprägniermittels im spezifischen Bereich (24, 112) umfasst, **dadurch gekennzeichnet, dass** erster und zweiter Schritt gleichzeitig durchgeführt werden.

2. Verfahren nach dem vorhergehenden Anspruch, **dadurch gekennzeichnet, dass** erster und zweiter Schritt wenigstens teilweise mit einer gleichen Vorrichtung (13, 102) durchgeführt werden.

3. Verfahren nach Anspruch 1 oder 2, **dadurch gekennzeichnet, dass** die Endlosbahn (22, 106) aus einem Vliesmaterial, insbesondere einer Baumwollbahn, besteht.

4. Verfahren nach Anspruch 1 oder 2, **dadurch gekennzeichnet, dass** die Endlosbahn (22, 106) aus einem Zellulosematerial, insbesondere einem Papierbogen, besteht.

5. Vorrichtung nach einem der vorhergehenden Ansprüche, **dadurch gekennzeichnet, dass** das Imprägniermittel eine Flüssigkeit ist.

6. Vorrichtung nach einem der Ansprüche 1 bis 4, **dadurch gekennzeichnet, dass** das Imprägniermittel eine Paste ist.

7. Zum Ausführen des Verfahrens nach einem der Ansprüche 1 bis 6 ausgebildete Vorrichtung (10, 100), **dadurch gekennzeichnet, dass** sie wenigstens ein erstes Mittel (13, 102) zum Zuschneiden eines spezifischen Bereichs (24, 112) einer aus einem Fasermaterial gebildeten Endlosbahn (22, 106) und wenigstens ein zweites Mittel (50, 116) zum Aufbringen eines Imprägniermittels im spezifischen Bereich (24, 112) umfasst, wobei erstes (13, 102) und zweites Mittel (50, 116) aus einem Teil bestehen.

8. Vorrichtung (10, 100) nach dem vorhergehenden Anspruch, **dadurch gekennzeichnet, dass** das zweite Mittel (50, 116) in Bezug auf das erste Mittel (13, 102) so angeordnet ist, dass es sich zum Zeitpunkt des Zuschneidens in Kontakt mit oder gegenüber dem spezifischen Bereich (24, 112) befindet.

9. Vorrichtung (10) nach Anspruch 7 oder 8, **dadurch gekennzeichnet, dass** das erste Mittel (13) aus einer mit einem vertieften oder erhabenen Motiv (18) versehenen Rolle besteht, deren Ränder (40) schneiden und allgemein den zuzuschneidenden spezifischen Bereich (24) abgrenzen, wobei die Endlosbahn (22) zwischen dieser Rolle (13) und einer glatten Rolle (12), der sogenannten Ambossrolle, zusammengedrückt wird, so dass das Zuschneiden erfolgt.

10. Vorrichtung (10) nach Anspruch 7 oder 8, **dadurch gekennzeichnet, dass** das erste Mittel aus einer auf einem Träger (14) montierten Klinge besteht.

11. Vorrichtung (10) nach dem vorhergehenden Anspruch, **dadurch gekennzeichnet, dass** die Klinge im Träger (14) beweglich ist.

12. Vorrichtung (10) nach Anspruch 10 oder 11, **dadurch gekennzeichnet, dass** der Träger (14) gekrümmt und vorzugsweise zylindrisch ist.

13. Vorrichtung nach Anspruch 10 oder 11, **dadurch gekennzeichnet, dass** der Träger eben ist.

14. Vorrichtung (100) nach Anspruch 7 oder 8, **dadurch gekennzeichnet, dass** das erste Mittel (102) eine Stanze ist, wobei die Endlosbahn (106) zwischen dieser Stanze (102) und einer von wenigstens einem Loch (108) durchbohrten Matrize (104), dessen Maße denen des zuzuschneidenden spezifischen Bereichs (112) entsprechen, zusammengedrückt wird, so dass das Zuschneiden durchgeführt wird.

15. Vorrichtung (10, 100) nach einem der Ansprüche 7 bis 14, **dadurch gekennzeichnet, dass** das zweite Mittel (50, 116) eine zum Spritzen des Imprägniermittels in Richtung der Endlosbahn (22, 106) ausgebildete Düse umfasst.

16. Vorrichtung (10) nach einem der Ansprüche 7 bis 14, **dadurch gekennzeichnet, dass** das zweite Mittel (50) einen zur Aufnahme einer bestimmten Menge von Imprägniermittel und zum Übertragen wenigstens eines Teils des Mittels auf die Bahn (22), wenn er gegen diese gedrückt wird, ausgebildeten absorbierenden Körper umfasst.

17. Vorrichtung (10,100) nach einem der Ansprüche 7 bis 14, **dadurch gekennzeichnet, dass** das zweite Mittel (50,102) einen zum Rückhalten einer bestimmten Menge von Imprägniermittel auf seiner Oberfläche und zum Übertragen wenigstens eines Teils des Mittels auf die Bahn (22,106), wenn er in Kontakt mit dieser gebracht wird, ausgebildeten nicht absorbierenden Körper umfasst.

## Claims

1. Method for the impregnation of a product consisting of fibrous material and formed from a continuous web (22, 106), the method comprising at least one first step of cutting out a specific zone (24, 112) of the said continuous web (22, 106) and at least one second step of applying an impregnation product to the said specific zone (24, 112), **characterized in that** the first and second steps are carried out simultaneously.

2. Method according to the preceding claim, **characterized in that** the first and second steps are carried out at least partially by means of one and the same apparatus (13, 102).

3. Method according to Claim 1 or 2, **characterized in that** the continuous web (22, 106) is formed from a non-woven material, in particular a cotton web.

4. Method according to Claim 1 or 2, **characterized in that** the continuous web (22, 106) is formed from a cellulose material, in particular a paper sheet.

5. Method according to any one of the preceding claims, **characterized in that** the impregnation product is a liquid.

6. Method according to any one of Claims 1 to 4, **characterized in that** the impregnation product is a paste.

7. Device (10, 100) intended for carrying out the method according to any one of Claims 1 to 6, **characterized in that** it comprises at least one first means (13, 102) for cutting out a specific zone (24, 112) of a continuous web (22, 106) formed from a fibrous material, at least one second means (50, 116) for applying an impregnation product to the said specific zone (24, 112), the first (13, 102) and second (50, 116) means being in one piece.

8. Device (10, 100) according to the preceding claim, **characterized in that** the second means (50, 116) is arranged in relation to the first means (13, 102) so as to be in contact with or opposite to the specific zone (24, 112) at the moment of the cut-out.

9. Device (10) according to either one of Claims 7 and 8, **characterized in that** the first means (13) consists of a roller provided with a recess or relief pattern (18), the edges (40) of which are cutting and substantially delimit the specific zone (24) to be cut out, the continuous web (22) being pressed between this roller (13) and a smooth roller (12), called an anvil roller, so as to carry out the cut-out.

10. Device (10) according to either one of Claims 7 and 8, **characterized in that** the first means consists of a blade mounted on a support (14).

11. Device (10) according to the preceding claim, **characterized in that** the blade is movable in the support (14).

12. Device (10) according to either one of Claims 10 and 11, **characterized in that** the support (14) is curved and is preferably cylindrical.

13. Device according to either one of Claims 10 and 11, **characterized in that** the support is plane.

14. Device (100) according to either one of Claims 7 and 8, **characterized in that** the first means (102) is a punch, the continuous web (106) being pressed between this punch (102) and a die (104) pierced with at least one hole (108), the dimensions of which correspond to those of the specific zone (112) to be cut out, so as to carry out the cut-out.

15. Device (10, 100) according to any one of Claims 7 to 14, **characterized in that** the second means (50, 116) comprises a nozzle capable of projecting the impregnation product in the direction of the continuous web (22, 106).

16. Device (10) according to any one of Claims 7 to 14, **characterized in that** the second means (50) comprises an absorbent body capable of containing a certain quantity of impregnation product and of transferring at least part of the said product onto the web (22) when it is pressed against the latter.

17. Device (10, 100) according to any one of Claims 7 to 14, **characterized in that** the second means (50, 102) comprises a non-absorbent body capable of retaining a certain quantity of impregnation product on its surface and of transferring at least part of the said product onto the web (22, 106) when it is put into contact with the latter.
